(19) Europäisches Patentamt / European Patent Office / Office européen des brevets

(11) **EP 3 928 837 A1**

(12) **EUROPEAN PATENT APPLICATION**
published in accordance with Art. 153(4) EPC

(43) Date of publication:
**29.12.2021 Bulletin 2021/52**

(21) Application number: **20760295.4**

(22) Date of filing: **21.02.2020**

(51) Int Cl.:
*A61Q 1/12* $^{(2006.01)}$       *A61K 8/02* $^{(2006.01)}$
*A61K 8/44* $^{(2006.01)}$       *A61K 8/73* $^{(2006.01)}$

(86) International application number:
**PCT/JP2020/007149**

(87) International publication number:
**WO 2020/171217 (27.08.2020 Gazette 2020/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.02.2019  JP 2019030775**

(71) Applicant: **AJINOMOTO CO., INC.
Chuo-ku
Tokyo 104-8315 (JP)**

(72) Inventor: **WAKUI, Wataru
Kawasaki-shi, Kanagawa 210-8681 (JP)**

(74) Representative: **Mewburn Ellis LLP
Aurora Building
Counterslip
Bristol BS1 6BX (GB)**

(54) **POWDER AND COSMETIC PREPARATION**

(57)   The present invention provides a powder which is configured such that one or more components among $N^{\varepsilon}$-lauroyl lysine and $N^{\varepsilon}$-octanoyl lysine are present on the surface of an elastic powder that has an elastic modulus of from $1 \times 10^5$ to $5 \times 10^{10}$ N/m$^2$.

EP 3 928 837 A1

**Description**

Technical Field

The present invention relates to a powder that improves a feel and a cosmetic preparation containing the powder.

Background Art

Conventionally, it has been known to blend a spherical polymer powder such as nylon powder (for example, Japanese Patent No. 2653990) or acrylic powder (for example, Japanese Patent No. 3027008) to a cosmetic preparation for the purpose of improving the feel and slipperiness at the time of application. In particular, a spherical nylon powder has a good characteristic feel and slipperiness at the time of application due to the rolling feel unique to the sphere, but has a problem that friction derived from the synthetic polymer resin is generated on the skin and a moist feel cannot be imparted.

On the other hand, a technique has been devised in which an N-mono long-chain acyl basic amino acid typified by $N^\varepsilon$-lauroyl lysine is blended as a cosmetic preparation powder into a cosmetic preparation to give a unique moist feel (Japanese Patent Application Publication No. Sho 61-10503). However, although this N-mono long-chain acyl basic amino acid has excellent elongation on the skin at the time of application, there is a problem that the feel and slipperiness due to the rolling feel as in spherical nylon powder at the time of application are poor.

$N^\varepsilon$-lauroyl lysine may be used as a surface treatment agent in order to improve the feel of talc (Japanese Patent Application Publication No. Hei 7-252113), but in order to produce a powder such as talc surface-treated with $N^\varepsilon$-lauroyl lysine imparted with high water repellency, adhesion, and transparency, it is necessary to once dissolve $N^\varepsilon$-lauroyl lysine in a strong alkali or a strong acid and precipitate it on the powder surface. The powders that can industrially actually be surface-treated with $N^\varepsilon$-lauroyl lysine have been limited to acid-resistant and alkaline powders having a special shape and a specific functional group, such as talc and mica. It has been impossible for powders such as corn starch to be surface-treated with $N^\varepsilon$-lauroyl lysine by such a method.

Summary of Invention

An object of the present invention is to provide a powder that is excellent in feel and slipperiness at the time of application, can impart a moist feel, and has no friction, and a cosmetic preparation containing the powder.

The present inventors have made earnest studies, and have found as a result that when corn starch powder is pulverized and mixed with one or two of $N^\varepsilon$-lauroyl lysine or $N^\varepsilon$-octanoyl lysine under specific conditions, a powder is prepared in which $N^\varepsilon$-lauroyl lysine or $N^\varepsilon$-octanoyl lysine, which is an inelastic powder, is present on the surface of corn starch, which is an elastic powder, and thus a powder that can solve the above problems can be obtained. The present invention has been completed based on this fact. Specifically, the present invention provides the following powders and cosmetic preparations.

[1] A powder comprising: an elastic powder having an elastic modulus of $1 \times 10^5$ to $5 \times 10^{10}$ N/m²; and one or more components of $N^\varepsilon$-lauroyl lysine and $N^\varepsilon$-octanoyl lysine present on a surface of the elastic powder.
[2] The powder according to [1], wherein the elastic powder has an elastic modulus of $1 \times 10^6$ to $1 \times 10^{10}$ N/m².
[3] The powder according to [1], wherein the elastic powder has an elastic modulus of $1 \times 10^7$ to $5 \times 10^8$ N/m².
[4] The powder according to [1], wherein the elastic powder has an elastic modulus of $1 \times 10^7$ to $3 \times 10^8$ N/m².
[5] The powder according to any one of [1] to [4], wherein a ratio of a total mass of the one or more components to a mass of the elastic powder is 1:9 to 4:6.
[6] The powder according to any one of [1] to [5], wherein a mean particle size is 5 to 25 $\mu$m, and a particle size at a cumulative frequency of 90% of all particles is 35 $\mu$m or less.
[7] The powder according to any one of [1] to [6], wherein a mean particle size is 10 to 20 $\mu$m, and a particle size at a cumulative frequency of 90% of all particles is 15 to 30 $\mu$m.
[8] The powder according to any one of [1] to [7], wherein the elastic powder is corn starch.
[9] The powder according to any one of [1] to [8], which is spherical.
[10] A cosmetic preparation comprising: the powder according to any one of [1] to [9].
[11] A powder obtained by pulverizing and mixing one or more of $N^\varepsilon$-lauroyl lysine and $N^\varepsilon$-octanoyl lysine with an elastic powder having an elastic modulus of $1 \times 10^5$ to $5 \times 10^{10}$ N/m².
[12] The powder according to [11], wherein a mean particle size is 5 to 25 $\mu$m, and a particle size at a cumulative frequency of 90% of all particles is 35 $\mu$m or less.

Brief Description of Drawings

Fig. 1 is an optical micrograph showing a particle image of corn starch.
Fig. 2 is an optical micrograph showing a particle image of the powder of the present invention.

Description of Embodiments

The powder of the present invention is a powder comprising: an elastic powder having an elastic modulus of $1 \times 10^5$ to $5 \times 10^{10}$ N/m$^2$; and one or more components of N$^\varepsilon$-lauroyl lysine and N$^\varepsilon$-octanoyl lysine present on a surface of the elastic powder. Here, the one or more components present on the surface of the elastic powder may mean that the one or more components are present on at least a part of the surface of the elastic powder through adhesion or the like, and the mode thereof is not particularly limited.

The elastic powder used in the present invention has an elastic modulus of $1 \times 10^5$ to $5 \times 10^{10}$ N/m$^2$, preferably $1 \times 10^6$ to $1 \times 10^{10}$ N/m$^2$, more preferably $1 \times 10^7$ to $5 \times 10^8$ N/m$^2$, and further preferably $1 \times 10^7$ to $3 \times 10^8$ N/m$^2$. By pulverizing and mixing such an elastic powder with one or more components of N$^\varepsilon$-lauroyl lysine and N$^\varepsilon$-octanoyl lysine under specific conditions, it is possible to prepare a powder comprising: the elastic powder; and one or more of the above components present on the surface of the elastic powder. The elastic powder includes corn starch, rice starch, potato starch, tapioca starch, wheat flour, barley flour, rye flour, pregelatinized starch, partially pregelatinized starch, polymethyl methacrylate, methyl methacrylate crosspolymer, polyacrylic acid, nylon-6, nylon-12, nylon-11, polyurethane, polyethylene, polypropylene, polystyrene, silicone resins, (vinyl dimethicone/methicone silsesquioxane) crosspolymer, sodium carboxymethyl starch, carmellose, carmellose sodium, carmellose calcium, croscarmellose sodium, crospovidone, low-substituted hydroxypropyl cellulose, methyl cellulose, ethyl cellulose, hypromellose, hydroxypropyl cellulose, hydroxypropyl methylcellulose phthalate, hydroxypropyl methylcellulose acetate succinate, carboxymethyl ethylcellulose, sodium carboxymethyl cellulose, hydroxyethyl cellulose, cellulose acetate phthalate, crystalline cellulose, polyethylene glycol, polyvinyl alcohol, polyvinylpyrrolidone, polyvinyl acetal diethylaminoacetate, aminoalkyl methacrylate copolymer E, aminoalkyl methacrylate copolymer RS, methacrylic acid copolymer L, methacrylic acid copolymer LD, methacrylic acid copolymer S, carboxyl vinyl polymers, arabic rubber, polystyrene/butadiene rubber, natural rubber, sodium alginate, propylene glycol alginate ester, agar, gelatin, tragacanth, xanthan gum, guar gum, polyarginine, polylysine, polyglutamic acid, ceresin, microcrystalline wax, porous silica, silica, and the like. The elastic powder is preferably corn starch. Any of these elastic powders may be used alone, or two or more of them may be mixed and used in combination.

The ratio of the total mass of the one or more components to the mass of the elastic powder is preferably 1:9 to 6:4, and more preferably 1:9 to 4:6. When the ratio is in such a range, it is possible to efficiently prepare a powder comprising: the elastic powder; and one or more components of N$^\varepsilon$-lauroyl lysine and N$^\varepsilon$-octanoyl lysine present on the surface of the elastic powder.

The powder of the present invention can be prepared by pulverizing and mixing the elastic powder and the one or more components with a ball mill using alumina balls having a diameter of 0.5 to 5 cm, for example. The mean particle size of the powder of the present invention is preferably 5 to 25 $\mu$m, and more preferably 10 to 20 $\mu$m. The particle size at a cumulative frequency of 90% of all particles of the powder of the present invention is preferably 35 $\mu$m or less, and more preferably 15 to 30 $\mu$m. By adjusting the particle size to these ranges, it is possible to improve the feel on the skin. The mean particle size and the particle size at a cumulative frequency of 90% of all particles can be measured using a laser diffraction/scattering type particle size distribution measuring device.

The powder of the present invention is preferably spherical. Here, "spherical" does not necessarily mean only a true sphere, but means that the cross-sectional shape of the particle is surrounded by a curved surface such as a circular shape, an elliptical shape, a substantially circular shape, or a substantially elliptical shape.

The cosmetic preparation of the present invention comprises: the above-described powder comprising: an elastic powder having an elastic modulus of $1 \times 10^5$ to $5 \times 10^{10}$ N/m$^2$; and one or more components of N$^\varepsilon$-lauroyl lysine and N$^\varepsilon$-octanoyl lysine present on a surface of the elastic powder.

In the cosmetic preparation of the present invention, as long as the effects of the present invention are not impaired, it is possible to appropriately blend ingredients usually used in cosmetic preparations such as powders, pigments, oils, ionic surfactants, nonionic surfactants, moisturizers, antioxidants, thickeners, film-forming agents, organic solvents, preservatives, chelating agents, and fragrances.

The formulation of the cosmetic preparation of the present invention is optional, and may be any formulation such as a solution type, a solubilization type, an emulsification type, and a powder dispersion type. The usage thereof includes skin cosmetic preparations such as lotions, milky lotions, creams, and packs, hair cosmetic preparations such as shampoos, rinses, hair creams, and hair sets, and makeup cosmetic preparations such as foundations, lipsticks, and eye shadows.

The pH of the cosmetic preparation of the present invention is not particularly limited to a specific range, but is generally 4.5 to 7. The pH of the cosmetic preparation of the present invention may be adjusted by adding a pH adjuster. The pH adjuster is not particularly limited, and includes, for example, lactic acid, citric acid, glycolic acid, succinic acid, tartaric acid, potassium carbonate, sodium hydrogen carbonate, ammonium hydrogen carbonate, sodium hydroxide, potassium hydroxide, triethanolamine, monoethanolamine, and the like. Any of these pH adjusters can be used alone or two or more of them may be used in combination.

Next, the present invention is described with reference to Examples and Comparative Examples, but the present invention is not limited thereto.

Examples

<Example 1>

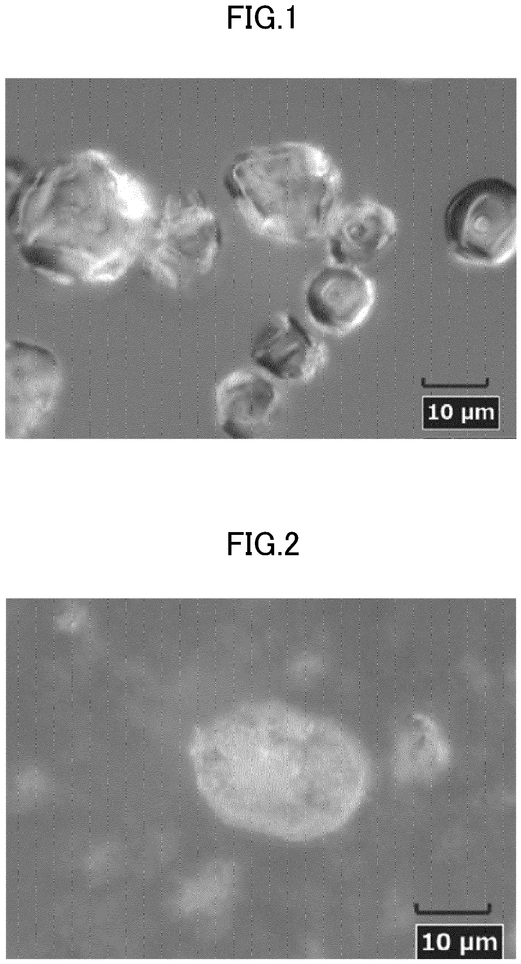

$N^{\varepsilon}$-lauroyl lysine (Ajinomoto Co., Inc., Amihope (registered trademark) LL) in an amount of 4g and corn starch (Nihon Shokuhin Kako Co., Ltd., Corn Starch W) in an amount of 16 g were measured in a ball mill container made of alumina having a volume of 1 L. In this container, 660 g of alumina balls having a diameter of 2.0 cm and 600 g of alumina balls having a diameter of 1.0 cm were placed, followed by pulverizing and mixing at 100 rpm for 15 hours to obtain a spherical powder having $N^{\varepsilon}$-lauroyl lysine adhering to the surface. To confirm that $N^{\varepsilon}$-lauroyl lysine adhered to the surface, it was confirmed by visual observation with an optical microscope (RH-2000 Digital Microscope manufactured by HIROX) that $N^{\varepsilon}$-lauroyl lysine adhered to the surface of spherical particles unique to corn starch (Figs. 1 and 2). The elastic modulus of the corn starch was $2.9 \times 10^8$ N/m$^2$. Note that the elastic modulus of the elastic powder was calculated by the following formula from the particle diameter measured one by one with an optical microscope, the cross-sectional area calculated from the particle diameter by regarding the particle cross section as a circle, the test force calculated by a micro compression tester (MCT-510 manufactured by Shimadzu Corporation), and the compressive displacement (deformation amount). The value of the elastic modulus is an average value of n = 5.

$$\text{Elastic modulus} = \sigma \div \varepsilon = (P \div A) \div (\lambda \div L)$$

$\sigma$: normal stress
P: test force
A: particle cross-sectional area
$\varepsilon$: strain
$\lambda$: compressive displacement (deformation amount)
L: particle diameter

In addition, when the arithmetic mean particle size was measured by the dry method with a laser diffraction/scattering type particle size distribution measuring device (LA-950 manufactured by HORIBA, Ltd.), the mean particle size was 12 $\mu$m, and the particle size at a cumulative frequency of 90% of all particles was 20 $\mu$m. Note that the arithmetic mean particle size is expressed by the following formula.

$$\text{Arithmetic mean particle size } (\mu m) = \Sigma\{q(J) \times X(J)\} \div \Sigma\{q(J)\}$$

J: particle size division number
q(J): frequency distribution value (%)
X(J): representative diameter ($\mu$m) of the J-th particle size range

<Example 2>

A spherical powder having $N^{\varepsilon}$-lauroyl lysine adhering to the surface was obtained in the same manner as in Example 1 except that the pulverizing and mixing time was changed to 5 hours. The mean particle size was 12 $\mu$m, and the particle size at a cumulative frequency of 90% of all particles was 19 $\mu$m.

<Example 3>

A spherical powder having $N^{\varepsilon}$-lauroyl lysine adhering to the surface was obtained in the same manner as in Example 1 except that the pulverizing and mixing time was changed to 64 hours. The mean particle size was 13 μm, and the particle size at a cumulative frequency of 90% of all particles was 22 μm.

<Example 4>

A spherical powder having Nᵋ-lauroyl lysine adhering to the surface was obtained in the same manner as in Example 1 except that the pulverizing and mixing time was changed to 120 hours. The mean particle size was 15 μm, and the particle size at a cumulative frequency of 90% of all particles was 25 μm.

<Comparative Example 1>

The particle size distribution of Nᵋ-lauroyl lysine (Ajinomoto Co., Inc., Amihope (registered trademark) LL) was measured. The mean particle size was 17 μm, and the particle size at a cumulative frequency of 90% of all particles was 26 μm.

<Comparative Example 2>

The particle size distribution of corn starch (Nihon Shokuhin Kako Co., Ltd., Corn Starch W) was measured. The mean particle size was 20 μm, and the particle size at a cumulative frequency of 90% of all particles was 30 μm.

<Comparative Example 3>

Nᵋ-lauroyl lysine (Ajinomoto Co., Inc., Amihope (registered trademark) LL) in an amount of 4 g and corn starch (Nihon Shokuhin Kako, corn starch W) in an amount of 16 g were measured into a glass vial having a volume of 50 mL, and were manually shaken 200 times, and the particle size distribution of the resultant was measured. The mean particle size was 21 μm, and the particle size at a cumulative frequency of 90% of all particles was 32 μm.

Table 1 collectively shows the mean particle sizes and the particle sizes at a cumulative frequency of 90% of all particles of the powders of Examples 1 to 4 and Comparative Examples 1 to 3. From Table 1, the mean particle sizes were reduced by pulverizing and mixing with a ball mill. In addition, from the results of Examples 1 to 4, the mean particle sizes increased as the pulverizing and mixing time increased. From this, it is considered that more Nᵋ-lauroyl lysine adheres to the particle surface by lengthening the pulverizing and mixing time.

Table 1

| Evaluation Item | Example 1 | Example 2 | Example 3 | Example 4 | Comparative Example 1 | Comparative Example 2 | Comparative Example 3 |
|---|---|---|---|---|---|---|---|
| Pulverizing and mixing Time (Hours) | 15 | 5 | 64 | 120 | Untreated | Untreated | Untreated |
| Mean Particle size (μm) | 12 | 12 | 13 | 15 | 17 | 20 | 21 |
| Particle size at Cumulative Frequency of 90% of All Particles | 20 | 19 | 22 | 25 | 26 | 30 | 32 |

<Example 5>

A spherical powder having Nᵋ-lauroyl lysine adhering to the surface was obtained in the same manner as in Example 1 except that 2 g of Nᵋ-lauroyl lysine and 18 g of corn starch were pulverized and mixed. The mean particle size was 14 μm, and the particle size at a cumulative frequency of 90% of all particles was 20 μm.

<Example 6>

A spherical powder having Nᵋ-lauroyl lysine adhering to the surface was obtained in the same manner as in Example 1 except that 8 g of N$^\varepsilon$-lauroyl lysine and 12 g of corn starch were pulverized and mixed. The mean particle size was 15 $\mu$m, and the particle size at a cumulative frequency of 90% of all particles was 18 $\mu$m.

<Example 7>

A spherical powder having N$^\varepsilon$-octanoyl lysine adhering to the surface was obtained in the same manner as in Example 1 except that N$^\varepsilon$-lauroyl lysine was changed to N$^\varepsilon$-octanoyl lysine (Ajinomoto Co., Inc., Amihope (registered trademark) OL). The mean particle size was 10 $\mu$m, and the particle size at a cumulative frequency of 90% of all particles was 18 $\mu$m.

<Example 8>

A spherical powder having N$^\varepsilon$-lauroyl lysine and N$^\varepsilon$-octanoyl lysine adhering to the surface was obtained in the same manner as in Example 1 except that 4 g of N$^\varepsilon$-lauroyl lysine was changed to a mixture of 2 g of N$^\varepsilon$-lauroyl lysine and 2 g of N$^\varepsilon$-octanoyl lysine (Ajinomoto Co., Inc., Amihope (registered trademark) OL). The mean particle size was 11 $\mu$m, and the particle size at a cumulative frequency of 90% of all particles was 18 $\mu$m.

<Test Example 1> Feel Evaluation of Spherical Powder

For the corn starch (Comparative Example 2), commercially available nylon particles (Toray, SP-500) (Comparative Example 4), as well as spherical powders of Examples 1, 5, 6, 7, and 8, sensory evaluation was performed by five specialized panelists on (1) unique good feel (rolling feel) due to rolling feel, (2) slipperiness at the time of application, (3) moist feel after application, (4) frictionlessness after application, and (5) adhesion to the skin. In the evaluation, each powder was applied to the inner part of the forearm of the panelist, and with the spherical powder of Comparative Example 2 as a comparison target, evaluation was performed based on the following evaluation criteria. For each evaluation item, the average value M of the evaluation points of the five panelists was calculated, and Table 2 shows the criteria "A" for the case of 0.5 < M ≤ 2.0, "B" for the case of 0 < M ≤ 0.5, "C" for the case of -0.5 < M ≤ 0, and "D" for the case of -2.0 ≤ M ≤ -0.5. Note that in the present invention, the rolling feel means that there is a feel in the powder like balls rolling on the skin.

<Evaluation Criteria>

(1) Rolling Feel

2 points: there is a comfortable rolling feel compared to the comparison target
1 point: there is a slightly comfortable rolling feel compared to the comparison target
0 points: there is a rolling feel to a similar extent compared to the comparison target
-1 points: there is a slight sliding feel compared to the comparison target
-2 points: there is a sliding feel compared to the comparison target

(2) Slipperiness at the Time of Application

2 points: there is a comfortable slipperiness compared to the comparison target
1 point: there is a slightly comfortable slipperiness compared to the comparison target
0 points: there is a slipperiness to a similar extent compared to the comparison target
-1 points: there is a slight stuck compared to the comparison target
-2 points: there is a stuck compared to the comparison target

(3) Moist Feel after Application

2 points: there is a comfortable moist feel compared to the comparison target
1 point: there is a slightly comfortable moist feel compared to the comparison target

0 points: there is a moist feel to a similar extent compared to the comparison target

-1 points: there is a slight dryness compared to the comparison target

-2 points: there is a dryness compared to the comparison target

(4) Frictionlessness after Application

2 points: a feel of comfortable smoothness continues compared to the comparison target

1 point: a feel of slight comfortable smoothness continues compared to the comparison target

0 points: a feel of smoothness continues to a similar extent compared to the comparison target

-1 points: there is a slight friction compared to the comparison target

-2 points: there is a friction compared to the comparison target

(5) Adhesion to the Skin

2 points: very high adhesion compared to the comparison target

1 point: slightly high adhesion compared to the comparison target

0 points: Adhesion to a similar extent compared to the comparison target

-1 points: slightly floating compared to the comparison target

-2 points: floating compared to the comparison target

Table 2

| Evaluation Item | Example 1 | Example 5 | Example 6 | Example 7 | Example 8 | Comparative Example 4 |
|---|---|---|---|---|---|---|
| Rolling Feel | A | A | A | A | A | B |
| Slipperiness at the Time of Application | B | B | A | B | B | B |
| Moist Feel after Application | A | B | A | A | A | C |
| Frictionlessness after Application | A | A | B | B | B | D |
| Adhesion to the Skin | B | B | B | A | A | C |

<Example 9> Preparation of Pressed Foundation

A solid foundation was produced as follows according to the formulation shown in Table 3.

Production method: In Table 3, the components (A) were mixed, and the components (B) that had been heat-dissolved were added and further uniformly mixed, which were sieved with a 150 $\mu$m-opening sieve to make the particle size uniform, filled in a gold plate, and compression molded.

Table 3

| Component | | | Example 4 |
|---|---|---|---|
| A | Talc, Dimethicone *1 | | 18.40 |
| | Mica, Dimethicone *2 | | 32.00 |
| | Mica, Dimethicone *3 | | 20.00 |
| | Titanium Oxide, Dimethicone *4 | | 8.00 |
| | Titanium Oxide, Stearic Acid A1 *5 | | 3.00 |
| | Zinc Oxide, Methicone *6 | | 2.00 |
| | Red Iron Oxide, Dimethicone *7 | | 0.40 |
| | Yellow Iron Oxide, Dimethicone *8 | | 1.00 |
| | Black Iron Oxide, Dimethicone *9 | | 0.15 |
| | N$^\varepsilon$-Lauroyl Lysine *10 | | 2.00 |
| | Methylparaben | | 0.05 |
| | Spherical Powder of Example 1 | | 2.00 |
| B | Dimethicone *11 | | 4.98 |
| | Polyglyceryl-2 Tetraisostearate *12 | | 3.00 |
| | Hexa(Hydroxystearate/Stearate/Rosinate) Dipentaerythrityl *13 | | 1.00 |
| | Mineral Oil *14 | | 2.00 |
| | Methylparaben | | 0.01 |
| | Tocopherol | | 0.01 |
| Total | | | 100.0 |

The numerical values represent contents in % by weight
*1 SA-Talc JA-46R Miyoshi Kasei
*2 SA-Sericite FSE Miyoshi Kasei
*3 SA-Mica Y-2300 Miyoshi Kasei
*4 SA-Titanium CR50 (100%) Miyoshi Kasei
*5 MT-100Z Tayca
*6 MZ-303S Tayca
*7 SA-Red R-516PS (100%) Miyoshi Kasei
*8 SA-Yellow LL-100P (100%) Miyoshi Kasei
*9 SA-Black BL-100PS (100%) Miyoshi Kasei
*10 Amihope (registered trademark) LL Ajinomoto Co., Inc.
*11 KF-96A-20cs Shin-Etsu Chemical
*12 Cosmol 44V Nisshin OilliO
*13 Cosmol 168ARV Nisshin OilliO
*14 Hycoal K-230 Kaneda

<Comparative Example 5>

A pressed foundation was prepared in the same manner as in Example 9 except that the "Spherical Powder of Example 1" in Table 3 of Example 9 was changed to nylon powder (Toray, SP-500).

<Comparative Example 6>

A pressed foundation was prepared in the same manner as in Example 9 except that the "Spherical Powder of Example 1" in Table 3 of Example 9 was changed to corn starch (Nihon Shokuhin Kako Co., Ltd., Corn Starch W).

<Test Example 2> Feel Evaluation of Pressed Foundation

For Comparative Examples 5 and 6 as well as Example 9, sensory evaluation was performed by five specialized panelists on (1) unique good feel (rolling feel) due to rolling feel at the time of application, (2) slipperiness at the time of application, (3) moist feel after application, (4) frictionlessness after application, and (5) adhesion to the skin. In the evaluation, each powder was applied to the inner part of the forearm of the panelist, and with the pressed foundation of Comparative Example 6 as a comparison target, evaluation was performed based on the following evaluation criteria. For each evaluation item, the average value M of the evaluation points of the five panelists was calculated, and Table 3 shows the criteria "A" for the case of $0.5 < M \leq 2.0$, "B" for the case of $0 < M \leq 0.5$, "C" for the case of $-0.5 < M \leq 0$, and "D" for the case of $-2.0 \leq M \leq -0.5$.

<Evaluation Criteria>

(1) Rolling Feel

2 points: there is a comfortable rolling feel compared to the comparison target
1 point: there is a slightly comfortable rolling feel compared to the comparison target
0 points: there is a rolling feel to a similar extent compared to the comparison target
-1 points: there is a slight sliding feel compared to the comparison target
-2 points: there is a sliding feel compared to the comparison target

(2) Slipperiness at the Time of Application

2 points: there is a comfortable slipperiness compared to the comparison target
1 point: there is a slightly comfortable slipperiness compared to the comparison target
0 points: there is a slipperiness to a similar extent compared to the comparison target
-1 points: there is a slight stuck compared to the comparison target
-2 points: there is a stuck compared to the comparison target

(3) Moist Feel after Application

2 points: there is a comfortable moist feel compared to the comparison target
1 point: there is a slightly comfortable moist feel compared to the comparison target
0 points: there is a moist feel to a similar extent compared to the comparison target
-1 points: there is a slight dryness compared to the comparison target
-2 points: there is a dryness compared to the comparison target

(4) Frictionlessness after Application

2 points: a feel of comfortable smoothness continues compared to the comparison target
1 point: a feel of slight comfortable smoothness continues compared to the comparison target
0 points: a feel of smoothness continues to a similar extent compared to the comparison target
-1 points: there is a slight friction compared to the comparison target
-2 points: there is a friction compared to the comparison target

(5) Adhesion to the Skin

2 points: very high adhesion compared to the comparison target

1 point: slightly high adhesion compared to the comparison target
0 points: Adhesion to a similar extent compared to the comparison target
-1 points: slightly floating compared to the comparison target
-2 points: floating compared to the comparison target

Table 4

| Evaluation Item | Example 9 | Comparative Example 5 |
|---|---|---|
| Rolling Feel | A | A |
| Slipperiness at the Time of Application | B | B |
| Moist Feel after Application | A | D |
| Frictionlessness after Application | B | C |
| Adhesion to the Skin | A | B |

**Claims**

1. A powder comprising: an elastic powder having an elastic modulus of $1 \times 10^5$ to $5 \times 10^{10}$ N/m$^2$; and one or more components of N$^\varepsilon$-lauroyl lysine and N$^\varepsilon$-octanoyl lysine present on a surface of the elastic powder.

2. The powder according to claim 1, wherein the elastic powder has an elastic modulus of $1 \times 10^6$ to $1 \times 10^{10}$ N/m$^2$.

3. The powder according to claim 1, wherein the elastic powder has an elastic modulus of $1 \times 10^7$ to $5 \times 10^8$ N/m$^2$.

4. The powder according to claim 1, wherein the elastic powder has an elastic modulus of $1 \times 10^7$ to $3 \times 10^8$ N/m$^2$.

5. The powder according to any one of claims 1 to 4, wherein a ratio of a total mass of the one or more components to a mass of the elastic powder is 1:9 to 4:6.

6. The powder according to any one of claims 1 to 5, wherein a mean particle size is 5 to 25 $\mu$m, and a particle size at a cumulative frequency of 90% of all particles is 35 $\mu$m or less.

7. The powder according to any one of claims 1 to 6, wherein a mean particle size is 10 to 20 $\mu$m, and a particle size at a cumulative frequency of 90% of all particles is 15 to 30 $\mu$m.

8. The powder according to any one of claims 1 to 7, wherein the elastic powder is corn starch.

9. The powder according to any one of claims 1 to 8, which is spherical.

10. A cosmetic preparation comprising: the powder according to any one of claims 1 to 9.

11. A powder obtained by pulverizing and mixing one or more of N$^\varepsilon$-lauroyl lysine and N$^\varepsilon$-octanoyl lysine with an elastic powder having an elastic modulus of $1 \times 10^5$ to $5 \times 10^{10}$ N/m$^2$.

12. The powder according to claim 11, wherein a mean particle size is 5 to 25 $\mu$m, and a particle size at a cumulative frequency of 90% of all particles is 35 $\mu$m or less.

# FIG.1

# FIG.2

## INTERNATIONAL SEARCH REPORT

| International application No. |
| --- |
| PCT/JP2020/007149 |

A. CLASSIFICATION OF SUBJECT MATTER
A61Q 1/12(2006.01)i; A61K 8/02(2006.01)i; A61K 8/44(2006.01)i; A61K 8/73(2006.01)i
FI: A61K8/44; A61K8/02; A61Q1/12; A61K8/73
According to International Patent Classification (IPC) or to both national classification and IPC

B. FIELDS SEARCHED

Minimum documentation searched (classification system followed by classification symbols)
A61Q1/12; A61K8/02; A61K8/44; A61K8/73

Documentation searched other than minimum documentation to the extent that such documents are included in the fields searched
　　　Published examined utility model applications of Japan　　　1922–1996
　　　Published unexamined utility model applications of Japan　　　1971–2020
　　　Registered utility model specifications of Japan　　　1996–2020
　　　Published registered utility model applications of Japan　　　1994–2020

Electronic data base consulted during the international search (name of data base and, where practicable, search terms used)
　　　JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
| --- | --- | --- |
| X | US 5662937 A (MCCUAIG, Dorothy) 02.09.1997 (1997-09-02) claims, column 2, lines 36-56, column 3, lines 47-50, example II | 1-12 |
| A | WO 99/44750 A1 (THE PROCTER & GAMBLE COMPANY) 10.09.1999 (1999-09-10) claims, examples I, II | 1-12 |

☐ Further documents are listed in the continuation of Box C. ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 April 2020 (15.04.2020) | 28 April 2020 (28.04.2020) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

| International application No. |
| --- |
| PCT/JP2020/007149 |

| Patent Documents referred in the Report | Publication Date | Patent Family | Publication Date |
| --- | --- | --- | --- |
| US 5662937 A | 02 Sep. 1997 | CA 2130967 A1 | |
| WO 99/44750 A1 | 10 Sep. 1999 | AU 2438099 A | |

Form PCT/ISA/210 (patent family annex) (January 2015)

**EP 3 928 837 A1**

**REFERENCES CITED IN THE DESCRIPTION**

**Patent documents cited in the description**

- JP 2653990 B
- JP 3027008 B
- JP 61010503 A
- JP 7252113 A